Europäisches Patentamt

European Patent Office

Office européen des brevets

(11) Publication number: 0 209 644
A1

# EUROPEAN PATENT APPLICATION

(21) Application number: 86105389.0

(51) Int. Cl.⁴: **A61M 5/14** , A61M 31/00

(22) Date of filing: 18.04.86

(30) Priority: 02.05.85 US 729860

(43) Date of publication of application:
28.01.87 Bulletin 87/05

(84) Designated Contracting States:
CH DE FR GB LI SE

(71) Applicant: IVAC CORPORATION
10300 Campus Pt. Drive
San Diego, CA 92121(US)

(72) Inventor: Maget, Henri J.R.
80 Arbuelo Way
Los Altos California 94022(US)

(74) Representative: Howden, Christopher Andrew
et al
FORRESTER & BOEHMERT
Widenmayerstrasse 4/I
D-8000 München 22(DE)

(54) Electrochemically driven drug dispenser.

(57) An improved drug dispenser (12 + 14) in which an electrochemical pump (12) is coupled through a bladder (32) or other suitable means to a drug-containing vessel (14) such that the pump's variable pressure source can be used to accurately control the delivery rate of small quantities of drugs into the human body. The drug dispenser (12 + 14) can be made small and implantable, has no moving parts which can wear out and break down and consumes little power from its power source. (24)

FIG. 1

EP 0 209 644 A1

## ELECTROCHEMICALLY DRIVEN DRUG DISPENSER

This invention relates generally to devices for the infusion of drugs into the human body, and, more particularly, to pump driven devices for the highly accurate infusion of small quantities of drugs.

The administration of drugs to a patient can be accomplished through a variety of methods, including gravity, mechanical pumps, pressurized gases and osmosis. A gravity dispenser utilizes the force of gravity to dispense a drug suspended above the patient. The delivery rate of the drug is adjusted by means of the pressure head, the height of the drug above the patient, and by a restriction of the outlet line leading to the patient. However, the delivery rate of the gravity dispenser is difficult to control accurately because of changes in the height of the drug as it is dispensed to the patient, changes in the posture of the patient and even variations in the back pressure of the patient's blood. More importantly, however, the patient is greatly restricted in mobility, since the drug must be constantly suspended in a container above the patient. A typical controller for adjusting the delivery rate of the gravity force dispenser is disclosed in U.S. Patent No. 4,300,552 to Cannon.

Other methods for administering drugs eliminate the need for suspending the drug above the patient and, therefore, greatly improve the mobility of the patient. Mechanical pump dispensers generally consist of an electrically-driven mechanical pump. These pumps provide accurate control of the delivery rate of the drug, but incorporate moving parts, which can wear out and break down. These pumps also con sume relatively large amounts of power. Other dispensers employ a pressurized gas to administer the drug to the patient. Generally, these dispensers are large and bulky, and not easily portable. Furthermore, regulation of the delivery rate is often difficult. Typical pressurized gas dispensers are disclosed in U.S. Patent No. 2,766,907 to Wallace, Jr. and U.S. Patent No. 4,237,881 to Beigler et al.

Osmosis driven dispensers depend on solutes that exhibit an osmotic pressure gradient against water. The delivery rate provided by these dispensers is determined by the type of solute used and, therefore, the delivery rate cannot be varied during operation. Representative osmotic dispensers are disclosed in U.S. Patent No. 3,995,632 to Nakano et al., U.S. Patent No. 4,034,756 to Higuchi et al. and U.S. Patent No. 4,439,196 to Higuchi. An electrically-controlled osmotic dispenser is disclosed in U.S. Patent No. 3,923,426 to Theeuwes.

Many patients require a continuous infusion of small quantities of drugs. Accordingly, there has been a need for a drug dispenser which is portable, can be miniaturized and therefore implanted, and be highly accurate in the delivery of small quantities of drugs.

The present invention resides in an electrochemically driven drug dispenser wherein an electrochemical pump provides a variable pressure source for the highly accurate control of the delivery rate of small quantities of drugs. The electrochemical pump is a device in which an electrochemically active gas, a gas susceptible to an oxidation/reduction reaction, is pumped from an upper chamber, across an electrolytic membrane, into a lower chamber. The lower chamber is operatively coupled to a drug-containing vessel in such a manner that, as the electrochemical gas is pumped into the lower chamber, the drug is likewise dispensed to the patient. Any barrier that imparts the pressure generated in the lower chamber to the drug may be employed for the coupling mechanism, such as a bladder or a sliding wall.

The significance of this invention is the high accuracy with which the delivery rate of small quantities of drugs can be controlled with a device that is small and implantable and has no moving parts. The rate of delivery of the drug is controlled by the rate of flow of the electrochemical gas across the membrane into the lower chamber, which, in turn, is controlled by the current drawn by the pump. Because the delivery rate is controlled by the electrical current, highly accurate delivery rates can be achieved, and constant or complex variable delivery rate schedules can be easily implemented in an analog or digital controller.

It will be appreciated from the foregoing that the present invention represents a significant advance in this field. Other features and advantages of the present invention will become apparent from the following detailed description, taken in conjunction with the accompanying drawings, which illustrate, by way of example, the principle of the invention.

FIGURE 1 is a sectional view of a drug dispenser employing a flexible expansible diaphragm and a delivery port;

FIGURE 2 is a sectional view of a drug dispenser employing a flexible expansible diaphragm and a drug specific barrier;

FIGURE 3 is a sectional view of a drug dispenser employing a sliding wall and a delivery port; and

FIGURE 4 is a graph showing the current-versus-time curve for a drug dispenser operating at a delivery rate of 0.01 ml/hr.

As shown in the drawings, the invention is concerned with a novel drug dispensing device. Many patients require the continuous infusion of small quantities of drugs and, therefore, a portable and small, and highly accurate and reliable drug dispenser has been required. Numerous present-day drug dispensers are designed for in-hospital use and are not easily portable. Others are portable and small, but may not provide the highly accurate control of the drug delivery rate that is required, or may have moving parts that can wear out and break down. Still others may have relatively large power requirements.

In accordance with the present invention, an electrochemical cell, in the form of a pump, provides a variable pressure source for the highly accurate control of the drug delivery rate, with no moving parts and with little power consumption. Moreover, the pump can be miniaturized for implantation. The electrochemical pump described in U.S. Patent No. 4,402,817 to the present inventor is typical of such an electrochemical pump. The electrochemical pump described in the present inventor's copending patent application entitled PLANAR MULTI-JUNCTION ELECTROCHEMICAL CELL is ideally suited for this pump application because of that cell's ability to provide a low flow rate in conjunction with a compatability with the voltage of the standard dry cell battery.

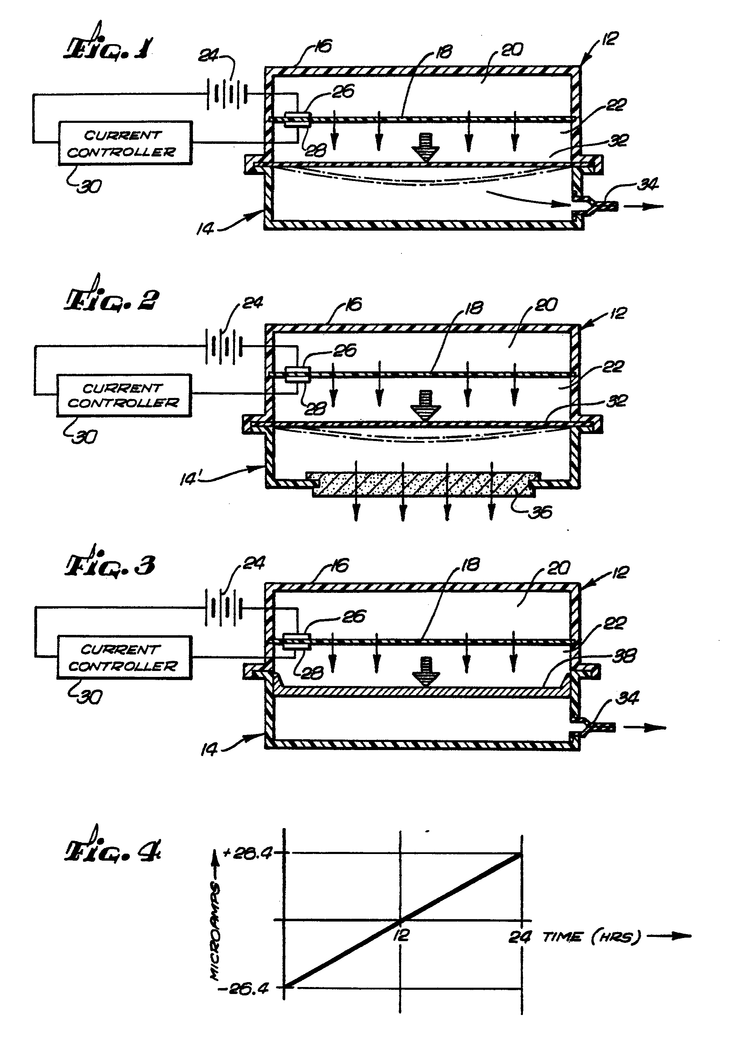

FIG. 1 illustrates one of the preferred em bodiments of the electrochemically driven drug dispenser. The drug dispenser comprises an electrochemical pump 12 and a drug-containing vessel 14. A gas-tight container 16 forms the pump chamber. An electrolytic membrane 18 disposed within the pump 12 divides the pump chamber into a supply chamber 20 and an outlet chamber 22. Upon the application of a voltage from a voltage source 24 to a pair of conductive electrodes 26 and 28, disposed on opposite surfaces of the electrolytic membrane 18, an electrochemically active gas contained in the supply chamber 20 is pumped across the membrane 18 into the outlet chamber 22. The flow rate across the membrane 18 is controlled by a current controller 30, which is located in series with the electrochemical pump 12 and the voltage source 24. A flexible and expansible diaphragm 32, which separates the outlet chamber 22 from the drug-containing vessel 14, expands into the drug-containing vessel 14 as the electrochemically active gas is pumped into the outlet chamber 22. The expansion of the diaphragm 32 squeezes the drug contained in the vessel 14 out through a delivery port 34. The delivery port 34 includes a check valve to prevent flow of the drug back into the vessel 14.

FIG. 2 illustrates a second preferred embodiment of the electrochemically driven drug dispenser. This embodiment employs the same electrochemical pump 12 and flexible diaphragm 32 as the first embodiment, but utilizes a slightly modified drug-containing vessel 14'. The vessel 14' has the delivery port 34 replaced with a drug specific barrier 36. The barrier 36 is a membrane specifically tailored for the drug to be delivered, and provides a fixed pressure drop for that drug across the membrane, while preventing back-flow. FIG. 3 illustrates a third preferred embodiment, but has the diaphragm 32 replaced with a sliding wall 38. The sliding wall 38 moves into vessel 14 as the electrochemically active gas is pumped into the outlet chamber 22 and squeezes the drug contained in the vessel 14 out through the delivery port 34.

The simple construction of the electrochemical pump 12 provides a device which has no moving parts and is highly reliable. The gas-tight container 16 can be composed of any material impervious to gas, such as metal, glass or plastic. The membrane 18, preferably a solid-type membrane, can be composed of any material containing dissociated functional groups capable of transporting either cations or anions. The electrodes 26 and 28 can be constructed of any material that is electrically conductive and acts as a catalyst in converting the gas molecules in the inlet chamber 20 to ions and reconverting those ions to gas molecules in the outlet chamber 22, in response to a voltage gradient applied across membrane 18.

The electrochemical pump 12 operates with any reduction/oxidation gas that is electrochemically reversibly active so as to react at the electrode 26 to produce ions, which will then migrate across the electrolytic membrane 18 and be reconverted at the electrode 28 into a molecular state. Hydrogen gas is one suitable example. At electrode 26 an anodic reaction occurs, represented by the equation:

$$H_2 \rightarrow 2H^+ + 2e^-$$

The hydrogen gas molecules in the supply chamber 28 are therefore converted into ions, which move across the electrolytic membrane. At electrode 28 a cathodic reaction occurs, represented by the equation:

$$2H^+ + 2e^- \rightarrow H_2$$

The hydrogen gas ions are therefore reconverted into hydrogen gas molecules and released into the outlet chamber 22. The net result is a flow of hydrogen gas from the supply chamber 20 to the outlet chamber 22. Other suitable gases include oxygen and air.

When the electrochemical pump 12 is at rest, diffusion of the electrochemical fluid across the membrane 18 results in an equalization of the pressures of the two chambers 20 and 22. Many materials, including electrolytic membranes, are susceptible to diffusion when a pressure differential exists across that material. Because the pressures of the two chambers 20 and 22 are equalized, the pump 12 must be activated prior to use. The pump 12 is activated by reversing the polarity of the voltage source 24 and pumping the gas contained in the outlet chamber 22 into the supply chamber 20. Supply chamber 20 will be fully charged when the diffusion across the membrane 18 due to the now higher pressure chamber 20 and the lower pressure chamber 22 equals the gas flow in the opposite direction due to the reverse-polarity pumping. For a fast charge and for a minimization of the work required by the voltage source 24, the activation of the pump 12 should be performed with an external power source. The drug-containing vessel 14 is then filled with a drug and the device is ready for use.

While fully charged, the pump will draw maximum reverse current, thereby pumping an equal amount of gas in the reverse direction as diffuses in the forward direction. To begin drug delivery, the reverse current being drawn by the pump 12 is reduced by the current controller 30 so that the pumping in the reverse direction is reduced below that of the diffusion in the forward direction. The result is some net gas flow into the outlet chamber 22.

The net gas flow into the outlet chamber 22, and hence the drug delivery rate, is controlled by the current controller 30. As the drug is delivered, the volume of the drug-containing vessel decreases and the volume of the outlet chamber 22 increases. The net gas flow into the outlet chamber 22 fills the increasing volume to maintain a constant pressure in that chamber. However, as gas is pumped from the supply chamber 20, the pressure in that chamber is reduced. The pressure differential between the two chambers is also reduced, which in turn causes a decrease in the diffusion from the supply chamber 20 to the outlet chamber 22. Accordingly, the current drawn by the pump 12 must be reduced to effect a reduction in the reverse pumping, which then offsets the decreased diffusion rate and maintains a constant net flow into the outlet chamber 22. This reduction in current is proportional to the reduction in diffusion across the membrane. The overall result is that the reverse current drawn by the pump must be linearly reduced over time by the current controller 30.

The linear reduction in reverse current drawn by the pump 12 will continue until the pressure differential between the two chambers is zero, which occurs when the current drawn by the pump 12 is zero. Forward pumping is then begun by a switch of the polarity of the voltage source 24 to the forward pumping direction. As the pressure in the supply chamber 20 is reduced below that of the constant pressure being maintained in the outlet chamber 22, the diffusion will be also reverse direction, being now from the outlet chamber 22 to the supply chamber 20. The diffusion rate will linearly increase, as well as the current required to maintain the constant pressure in the outlet chamber 22, until the diffusion from the outlet chamber 22 to the supply chamber 20 equals the maximum pumping in the forward direction. At this point, the drug vessel 26 must be refilled and the pump 12 re-activated.

In most cases, diffusion rates are very predictable, the rate being dependent on such variables as the type of membrane material, the membrane thickness and the type of gas. Therefore, the diffusion rate can be readily determined, which in turn allows for a straightforward and highly accurate calculation of the current required for a specific drug delivery rate. The net flow of the gas across the membrane, including both the pump flow and the diffusion flow, can be expressed by the relation:

$$R = \left[ \frac{P_e \times S_m \times (P_U - P_L)}{d} + K \times I \right] \times \frac{1}{P_L} \quad (1)$$

where R = the net flow rate,

$P_e$ = the permeability of the membrane,

d = the membrane thickness,

K = the pump flow rate constant,

I = the electrical current,

$P_U$ = the pressure in the upper chamber,

$P_L$ = the pressure in the lower chamber,

$S_m$ = the membrane area,

$V_U$ = the volume of the upper chamber,

and $V_L$ = the volume of the lower chamber.

If a constant delivery rate is desired, the pressure in the outlet chamber 22 ($P_L$) must remain constant. If a fixed supply chamber volume $V_U$ is used, then:

$$P_L \times V_L(t) + P_U(t) \times V_U = \text{Constant,} \quad (2)$$

where only $V_L$ and $P_U$ are functions of time.

Taking the derivative with respect to time:

$$P_L \times \frac{dV_L(t)}{dt} + V_U \times \frac{dP_U(t)}{dt} = 0 \quad (3)$$

But $\dfrac{dV_L(t)}{dt}$ is merely R, the net flow rate across the membrane.

Substituting R and rearranging equation (3):

$$- \frac{dP_U(t)}{dt} = \frac{P_L \times R}{V_U} \quad (4)$$

Integrating:

$$- P_U(t) + P_U(0) = \frac{P_L \times R \times t}{V_U} \quad (5)$$

Combining equations (1) and (5) yields the expression for the required current as a function of time:

$$I = \frac{R \times P_L}{K} + \frac{P_e \times S_m}{K \times d} \left[ P_L \left( \frac{R \times t}{V_U} + 1 \right) - P_U(0) \right] \quad (6)$$

Inserting the known constants into equation (6) yields a first order linear equation as a function of time. Such an equation can be implemented quite easily in any simple digital or analog controller.

For example, the controller for a typical electrochemically-driven drug dispenser, having a membrane with a surface area ($S_m$) of 1.0cm², a thickness (d) of 0.025 cm and a hydrogen permeability ($P_e$) of $6.25 \times 10^{-7}$ cm³-cm/cm²sec atm; a constant pressure in the lower chamber of 1.1 atmospheres; an upper chamber with a volume - ($V_U$) of 1 ml and containing hydrogen compressed initially ($P_U(0)$) to 1.354 atmospheres; and designed for delivering a drug at a rate of 0.01 ml/hr over a time period of 24 hours, would operate with the equation:

I = 2.2t-26.4 microamps.

A linear variation of the electrical current from -26.4 microamps to +26.4 microamps over the 24 hour period results, as shown in FIG. 4.

Further refinements to this electrochemically driven drug dispenser include provision in the current controller for non-linear current variations to permit scheduling the drug delivery rate as a function of time. Another refinement includes the addition of a flow rate sensor to the discharge port, to provide a feedback signal for even more accurate control of the delivery rate.

From the foregoing, it will be appreciated that an electrochemically driven drug dispenser provides an extremely accurate device for administering small amounts of a drug to a patient. Although several embodiments of the invention have been shown and described, it will be apparent that other adaptations and modifications can be made without departing from the spirit and scope of the invention.

The features disclosed in the foregoing description, in the following claims and/or in the accompanying drawings may, both separately and in any combination thereof, be material for realising the invention in diverse forms thereof.

## Claims

1. An electrochemically driven drug dispenser, comprising:

a drug-containing vessel having discharge means; and

an electrochemical pump operatively coupled to the drug-containing vessel, wherein the electrochemical pump is employed to pump an electrochemically active gas and pressurize a drug in the drug-containing vessel in order to dispense the drug through the discharge means.

2. The dispenser as defined in claim 1, wherein the electrochemical pump includes:

a container;

an electrolytic membrane disposed within the container to form a first and second chamber, the membrane being used to transport the electrochemically active gas after being oxidized;

a pair of electrodes disposed on either side of and in contact with the electrolytic membrane; and

means for supplying an electrical voltage to the electrodes.

3. The dispenser as defined in claim 2, wherein:

the discharge means includes a delivery port through which the drug is delivered, the delivery port including a check valve.

4. The dispenser as defined in claim 3, and further comprising:

a flexible and expansible diaphragm disposed between the second chamber and the drug-containing vessel.

5. The dispenser as defined in claim 4, wherein:

the diaphragm includes a bellows structure.

6. The dispenser as defined in claim 3, and further comprising:

a sliding wall disposed between the second chamber and the drug-containing vessel.

7. The dispenser as defined in claim 2, wherein:

the discharge means includes a membrane specifically tailored for the release of the fluid from the drug-containing vessel.

8. The dispenser as defined in claim 4, and further comprising:

controller means for regulating the electrical current drawn by the pump, the controller means being disposed in series with the means for supplying a voltage.

9. The dispenser as defined in claim 8, wherein:

the controller means further includes means for varying the electrical current linearly as a function of time.

10. The dispenser as defined in claim 8, wherein:

the controller means further includes means for varying the electrical current non-linearly as a function of time.

11. The dispenser as defined in claim 8, and further comprising:

an electrochemically active material disposed within the first and second chambers.

12. The dispenser as defined in claim 11, wherein:

the electrochemically active material includes hydrogen gas.

13. The dispenser as defined in claim 11, wherein:

the electrochemically active material includes oxygen gas.

FIG.1

FIG.2

FIG.3

FIG.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 195 461 (SIEMENS)<br>* Page 8, line 36 - page 9, line 37; figure 3 * | 1-5,7 | A 61 M 5/14<br>A 61 M 31/00 |
| | --- | | |
| X | BIOMEDIZINISCHE TECHNIK, vol. 22, July/August 1977, pages 169-173; G. LUFT et al.: "Elektroosmotische Pumpe zur gleichmässigen, gesteuerten oder geregelten Medikamentendosierung"<br>* Page 171, paragraph 2.2.2; figure 3 * | 1-5,7-13 | |
| | --- | | |
| A | US-A-3 995 632 (NAKANO)<br>* Figures * | 6 | |
| | --- | | TECHNICAL FIELDS SEARCHED (Int. Cl.4) |
| A | US-A-4 340 048 (ECKENHOFF)<br>* Figure 3 * | 1 | A 61 M |
| | ----- | | |

The present search report has been drawn up for all claims

| Place of search | Date of completion of the search | Examiner |
|---|---|---|
| THE HAGUE | 18-07-1986 | EHRSAM F.J.A. |